# EUROPEAN PATENT SPECIFICATION

(11) **EP 0 945 151 B1**
(45) Date of publication and mention of the grant of the patent: **04.12.2002**
(21) Application number: 99101558.7
(22) Date of filing: 28.01.1999
(51) Int. Cl.: A61M 16/14, A61M 16/18

(54) **Dosing device**
Dosiervorrichtung
Dispositif doseur

(30) Priority: 27.03.1998 SE 9801074
(43) Date of publication of application: 29.09.1999
(73) Proprietor: Siemens-Elema AB, 171 95 Solna (SE)
(72) Inventor: Wallin, Sten, 126 35 Hägersten (SE)

(56) References cited:
- EP-A1- 0 545 567
- US-A- 4 038 980

## Description

The present invention relates to a dosing device and particular to one useable in an anaesthesia machine or ventilator to regulate the concentration of a liquid vapour, such as an anaesthetic vapour, in a carrier gas component of a breathing gas.

It is common to administer liquid anaesthetic agents to a patient as a gas. This is done by vaporising the liquid and then carrying the vaporised anaesthetic to the patient in a carrier gas. It is important that the amount of anaesthetic agent is accurately controlled and this is generally achieved in one of two ways; either by controlling the flow rate of the carrier gas through a reservoir of liquid anaesthetic or by controlling the dose of liquid anaesthetic to be supplied for vaporisation. If a fault occurs in a device operating in the first way then relatively high doses can be taken from the reservoir. Even if other security measures for the patient interfere, the patient may still be exposed to quantities that could cause discomfort, and in the worst case even be harmful or lethal.

A known dosing device for controlling the dose of liquid anaesthetic to be supplied for vaporisation is described in WO 92/19303 (BOC Group Plc) and comprises a variable volume pump which can be operated to define the dose to be supplied by varying the volume in a controlled manner using a stepper motor. However the device demands that the pump and stepper motor are manufactured to a high mechanical tolerance, which adds to the cost of such a device as well as to the manufacturing difficulties. Additionally the generation of an intermittent flow, particularly of low flow rate, may be problematic as this requires accurate control of the pump volume over the majority of its stroke length.

It is an aim of the present invention to provide a dosing device in which the need for metering variations of pump volume is obviated.

This is achieved by a device according to and characterised by claim 1. In this device the flow of the liquid to be vaporised is monitored directly using a flow meter, the output of which operates a flow control means, for example a variable flow restriction, to regulate the supply of one or both of the liquid and carrier gas. Thus the need to determine and control exact pump chamber volume changes is removed and the dose may be supplied using a less sophisticated and relatively inexpensive overpressure and on-off valve arrangement.

Moreover, as flow control may be more readily achieved than volume control and as the supply is directly controlled from the actual liquid flow a controllable intermittent dose can be more readily achieved.

Preferably the flow meter comprises a pressure sensor adapted to provide a measure of the flow from the pressure exerted by incident liquid. Such a sensor is much better suited to the measurement of the relatively small flows that may be present in the dosing device than will be, for example, resistance type flow meters. Moreover, such a pressure sensor may be readily constructed, using current technology, to present little obstruction to the overall fluid flow path.

Usefully, the dosing device may comprise a guide means, such as a capillary tube, for directing substantially all of the flowing liquid on to the flow meter. This has the advantage, particularly at low doses, that the signal from the flow meter is a direct measure of the total amount of flowing liquid. Moreover, the capillary tube, because of its dimensions, may provide a substantially laminar liquid flow onto the sensor. Thus a more accurate flow measurement may be made as the flow characteristics are more predictable.

An embodiment of the invention will now be described, by way of example only, with reference to the drawing of the accompanying figure of which:

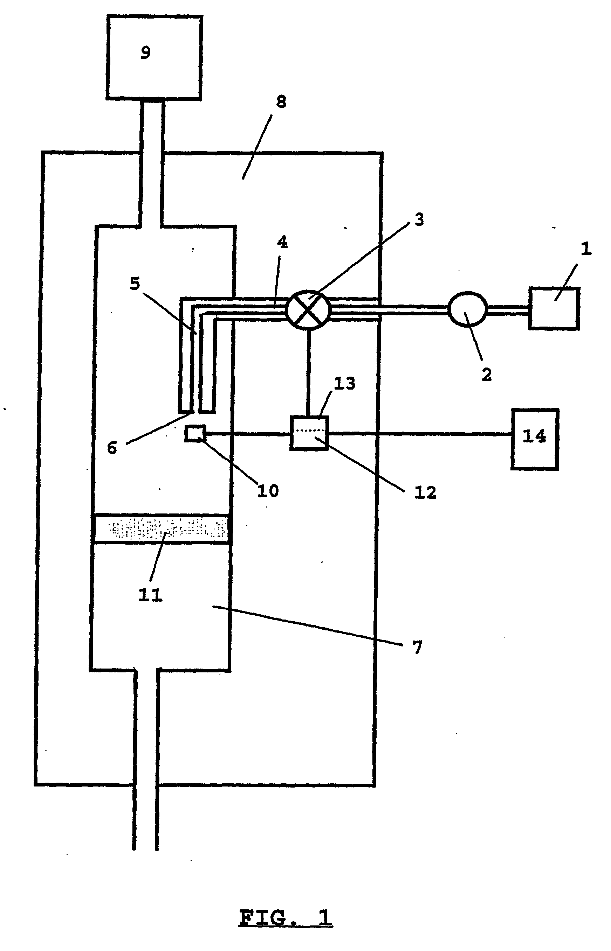

Figure 1 shows a representation of a dosing device according to the present invention.

Considering Figure 1, liquid anaesthetic from a liquid source 1 is pumped, using a pump 2, via an automatically controllable on-off valve 3 into an inlet 4 of a capillary tube 5. The tube 5 also being formed with an outlet 6 sited in a gas channel 7 of a vaporiser 8. A cylinder 9 of gas is also connected to the gas channel 7 and flows past the outlet 6 of the capillary tube 5 to carry the liquid anaesthetic which is then vaporised in this so called "carrier gas" before inspiration by a patient.

A pressure sensor type flow meter 10 is also placed within the gas channel 7 down stream of the outlet 6 of the capillary tube 5 so that substantially all of the liquid flowing from the capillary 5 is incident upon and is registered by the pressure sensor of the flow meter 10 before vaporisation. Additionally, the vaporiser may contain a sinter filter 11, downstream of the flow meter 10, which is well known in the art and comprises a large number of capillary through paths (for example created by the close packing of spheres which may constitute the filter 11) that create forces on the liquid to enhance its vaporisation.

A control means comprising the valve 3 and a valve controller 12 is operably connected to the flow meter 10 which outputs a signal to the controller 12 representative of the flow rate of the liquid anaesthetic measured by the meter 10. The controller 12 comprises a suitably programmed microprocessor means 13 and a timer, which here is a digital timer implemented in the microprocessor means, which acts to measure the time elapsed since the valve 3 was opened. The controller 12 is adapted to calculate from the input flow rate and from the elapsed time the quantity of liquid delivered into the gas channel 7. The controller 12 is further adapted to provide control signals to open and close the valve 3, thereby regulating the eventual concentration of anaesthetic vapour in the carrier gas, and operates to close the valve 3 when a predetermined (either input as a fixed value into a memory store of the microprocessor when the device is manufactured or input as a variable value from a user interface 14, such as a conventional keypad) dose has been delivered.

Preferably the meter 10 and the capillary tube 5 are arranged to minimise "dead space" between the valve 3 and the meter 10 from which space liquid will continue to be supplied after the valve 3 is closed and which therefore represents a minimum dose.

While the above embodiment of the present invention has been described as a possible implementation of that invention those skilled in the art will appreciate that variations thereof, such as for example additionally or alternatively placing a valve, the operation of which is controlled dependent on the output of the flow meter, towards the outlet of the carrier gas supply; or for example regulating the liquid flow by controlling the operation of the pump used to supply liquid to be vaporised in dependence of the measured liquid flow, will be possible while still falling within the scope of the claimed invention.

## Claims

1. A dosing device for regulating the concentration in a carrier gas of a vaporous agent derived from a liquid source (1), the device comprising control means (3,12) connectable to one or both sources of liquid (1) and carrier gas (9) and operable to automatically regulate the supply of one or both of the liquid and the carrier gas **characterised in that** the device further comprises a flow meter (10) in the flow path of the liquid supplied for vaporisation and adapted to generate an output signal proportional to the flow of that liquid; and **in that** the control means (3,12) is adapted to receive the output signal and to regulate the supply of one or both of the liquid and the carrier gas.

2. A dosing device as claimed in claim 1 **characterised in that** the control means comprises a variable flow restriction (3) operable to vary the supply of liquid for vaporisation dependent on the output signal.

3. A dosing device as claimed in claim 1 or claim 2 **characterised in that** there is additionally provided guide means (5) positionable to provide a flow path for directing the liquid flow towards the flow meter (10) with a cross sectional area sized to ensure that substantially all of the liquid is incident on a sensitive surface of the meter (10).

4. A dosing device as claimed in claim 3 **characterised in that** the guide means comprises a capillary tube (5), operably connected to the control means (3,12) to control the liquid flow there through, having an inlet (4) communicable with the liquid source and an outlet (6) communicable with a vaporiser (8).

5. A dosing device as claimed in claim 4 **characterised in that** the flow meter (10) is disposed proximal the outlet (6) of the tube (5).

6. A dosing device as claimed in any of the previous claims **characterised in that** the flow meter (10) comprises a pressure sensor and is adapted to determine the liquid flow from the pressure exerted on the sensor by the flowing liquid.

7. A dosing device as claimed in any previous claim **characterised in that** the control means (3,12) is adapted to regulate only the supply of liquid.

## Patentansprüche

1. Eine Dosiervorrichtung zur Regelung der Konzentration eines von einer Flüssigkeitsquelle (1) stammenden dampfartigen Mittels in einem Trägergas, wobei die Vorrichtung ein Steuermittel (3, 12) aufweist, das mit einer oder beiden Quelle(n) der Flüssigkeit (1) und des Trägergases (9) verbindbar und betätigbar ist, um automatisch die Zufuhr der Flüssigkeit oder des Trägergases oder beider zu regeln, **dadurch gekennzeichnet, dass** die Vorrichtung weiterhin einen Flussmesser (10) in dem Flussweg der zum Verdampfen zugeführten Flüssigkeit aufweist, der angepasst ist, ein Ausgangssignal zu erzeugen, das proportional zum Fluss der Flüssigkeit ist; und dass das Steuermittel (3, 12) angepasst ist, das Ausgangssignal zu empfangen und die Zufuhr der Flüssigkeit oder des Trägergases oder beider zu regeln.

2. Eine Dosiervorrichtung nach Anspruch 1, **dadurch gekennzeichnet, dass** das Steuermittel eine variable Flussbegrenzung (3) aufweist, die betätigbar ist, um die Zufuhr von Flüssigkeit zum Verdampfen abhängig von dem Ausgangssignal zu variieren.

3. Eine Dosiervorrichtung nach Anspruch 1 oder Anspruch 2, **dadurch gekennzeichnet, dass** zusätzlich Führungsmittel (5) vorgesehen sind, die so positionierbar sind, dass sie einen Flussweg schaffen, durch den der Flüssigkeitsfluss mit einer so bemessenen Querschnittsfläche in Richtung auf den Flussmesser (10) gerichtet wird, dass im wesentlichen die gesamte Flüssigkeit auf die sensitive Oberfläche des Messers (10) auftrifft.

4. Eine Dosiervorrichtung nach Anspruch 3, **dadurch gekennzeichnet, dass** die Führungsmittel ein Kapillarrohr (5) aufweisen, das wirkungsmäßig mit dem Steuermittel (3, 12) verbunden ist, um den Flüssigkeitsfluss durch dieses zu steuern, wobei das Kapillarrohr einen Einlass (4), der mit der Flüssigkeitsquelle in Verbindung bringbar ist, und einen Auslass (6), der mit einem Verdampfer (8) in Verbindung bringbar ist, hat.

5. Eine Dosiervorrichtung nach Anspruch 4, **dadurch gekennzeichnet, dass** der Flussmesser (10) in der Nähe des Auslasses (6) des Rohrs (5) angeordnet ist.

6. Eine Dosiervorrichtung nach einem der vorhergehenden Ansprüche, **dadurch gekennzeichnet, dass** der Flussmesser (10) einen Drucksensor aufweist und angepasst ist, den Flüssigkeitsfluss aus dem Druck abzuleiten, den die fließende Flüssigkeit auf den Sensor ausübt.

7. Eine Dosiervorrichtung nach einem der vorhergehenden Ansprüche, **dadurch gekennzeichnet, dass** das Steuermittel (3, 12) angepasst ist, nur die Zufuhr der Flüssigkeit zu regeln.

## Revendications

1. Dispositif de dosage pour réguler la concentration dans un gaz porteur d'un agent sous forme de vapeur dérivé d'une source liquide (1), le dispositif comportant les moyens (3, 12) de commande pouvant être connectés à une source de liquide (1) ou de gaz (9) porteur ou aux deux sources de liquide (1) et de gaz (9) porteur, et pouvant fonctionner pour réguler automatiquement l'alimentation en l'un ou les deux du liquide et du gaz porteur, **caractérisé en ce que** le dispositif comporte en outre un dispositif (10) de mesure de débit dans le trajet d'écoulement du liquide fourni pour la vaporisation et conçu pour produire un signal de sortie proportionnel au débit de ce liquide ; et **en ce que** les moyens (3, 12) de commande sont conçus pour recevoir le signal de sortie et réguler l'alimentation en l'un ou les deux du liquide et du gaz porteur.

2. Dispositif de dosage suivant la revendication 1, **caractérisé en ce que** les moyens de commande comportent une restriction (3) à l'écoulement variable qui peut fonctionner pour modifier l'alimentation en liquide pour la vaporisation en fonction du signal de sortie.

3. Dispositif de dosage suivant la revendication 1 ou 2, **caractérisé en ce qu'**il est prévu en outre des moyens (5) de guidage pouvant être positionnés pour obtenir un trajet d'écoulement pour diriger l'écoulement de liquide en direction du dispositif (10) de mesure de débit avec une aire en section transversale dimensionnée pour garantir que sensiblement tout le liquide est incident sur une surface sensible du dispositif (10) de mesure de débit.

4. Dispositif de dosage suivant la revendication 3, **caractérisé en ce que** les moyens de guidage comportent un tube (5) capillaire, connecté de manière fonctionnelle aux moyens (3, 12) de commande pour commander l'écoulement de liquide qui y passe, ayant une entrée (4) pouvant communiquer avec la source de liquide et une sortie (6) pouvant communiquer avec un vaporisateur (8).

5. Dispositif de dosage suivant la revendication 4, **caractérisé en ce que** le dispositif (10) de mesure de débit est disposé à proximité ou proximal à la sortie (6) du tube (5).

6. Dispositif de dosage suivant l'une quelconque des revendications précédentes, **caractérisé en ce que** le dispositif (10) de mesure de débit comporte un capteur de pression et est conçu pour déterminer le débit de liquide à partir de la pression exercée sur le capteur par le liquide qui s'écoule.

7. Dispositif de dosage suivant l'une quelconque des revendications précédentes, **caractérisé en ce que** les moyens (3, 12) de commande sont conçus pour réguler uniquement l'alimentation en liquide.
